(19) Europäisches Patentamt
European Patent Office
Office européen des brevets

(11) EP 4 517 762 A1

(12) **EUROPEAN PATENT APPLICATION**
published in accordance with Art. 153(4) EPC

(43) Date of publication:
05.03.2025 Bulletin 2025/10

(21) Application number: 22940148.4

(22) Date of filing: 27.04.2022

(51) International Patent Classification (IPC):
$G16C\ 20/10$ (2019.01)    $G16C\ 20/80$ (2019.01)

(52) Cooperative Patent Classification (CPC):
G06F 30/20; G16C 20/10; G16C 20/30;
G16C 20/70; G16C 20/80

(86) International application number:
PCT/JP2022/019088

(87) International publication number:
WO 2023/209869 (02.11.2023 Gazette 2023/44)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: FUJITSU LIMITED
Kawasaki-shi, Kanagawa 211-8588 (JP)

(72) Inventor: MITSUI, Takashi
Tokyo 105-7123 (JP)

(74) Representative: Hoffmann Eitle
Patent- und Rechtsanwälte PartmbB
Arabellastraße 30
81925 München (DE)

(54) **STRUCTURE EXTRACTION PROGRAM, STRUCTURE EXTRACTION METHOD, AND INFORMATION PROCESSING DEVICE**

(57) Molecular configuration samples, which reflect highly frequent intermolecular interactions, are extracted from the results of a molecular simulation.

An information processing apparatus (10) acquires molecular configuration samples (13, 14, ...). The information processing apparatus (10) generates, based on distances between pairs of the atoms in the plurality of molecular configuration samples (13, 14, ...), a score function (15) that calculates a weight for a pair of atoms that belong to different molecules out of the plurality of atoms, the score function including a parameter (16) indicating a distance threshold. The information processing apparatus (10) determines a specific value to be set in the parameter (16), based on a change in the weight calculated by the score function (15) in response to a change in a value of the parameter (16). The information processing apparatus (10) determines a priority order (17) of the plurality of molecular configuration samples (13, 14, ...) based on a specific weight calculated by the score function (15) using the specific value and distances between the pairs of atoms in each of the plurality of molecular configuration samples (13, 14, ...).

FIG. 1

**Description**

Technical Field

The embodiments discussed herein relate to a structure extraction program, a structure extraction method, and an information processing apparatus.

Background Art

Molecular simulation is one type of computer simulation. Molecular simulation is sometimes used to analyze intermolecular interactions, which indicate the attractive forces that different molecules apply to each other. Intermolecular interactions are analyzed in various fields, such as life sciences and material development.

As one example, in a molecular dynamics simulation, the interactive forces that act on each atom are calculated based on equations of motion while time advances in small increments, and the position and velocity of each atom are iteratively updated. As one example, a Monte Carlo simulation randomly generates molecular configuration samples according to a probability distribution based on the Boltzmann factor, and calculates expected values of physical properties from a plurality of molecular configuration samples. The Boltzmann factor indicates a weight that is inversely proportional to the energy of a molecular configuration.

A simulation apparatus that reproduces chemical changes, which accompany a rearrangement of covalent bonds in a large-scale atomic ensemble, by way of a molecular dynamics simulation has been proposed. A structure determining method has also been proposed that generates three-dimensional structures of dynamic organic molecules with interatomic bonds with variable angles and simulates changes in the three-dimensional structures in an ensemble of dynamic organic molecules.

Citation List

Patent Literature

[PTL1] Japanese Laid-open Patent Publication No. 2006-190234 [PTL2] International Publication Pamphlet No. WO2009/034297

Summary of Invention

Technical Problem

A molecular simulation generates a plurality of molecular configuration samples during processing. As one example, a molecular dynamics simulation generates a plurality of molecular configuration samples corresponding to a plurality of points in time. As another example, a Monte Carlo simulation randomly generates a plurality of molecular configuration samples. To assist in understanding the simulation results, it is desirable for an information processing apparatus to visualize and display some out of a plurality of molecular configuration samples (as one example, any one of the molecular configuration samples).

When doing so, it is important to determine which molecular configuration sample is to be extracted from the generated plurality of molecular configuration samples. The molecular configuration samples generated during a simulation process may include molecular configuration samples that do not reflect intermolecular interactions that appear with a high frequency. As one example, even when a group of atoms contained in one molecule and a group of atoms contained in another molecule are close to each other in a given molecular configuration sample, such proximity may be due to chance and not based on an intermolecular interaction that occurs with high frequency. Molecular configuration samples of that kind are likely to be less useful in understanding the simulation results.

For this reason, in one aspect, it is an object of the present disclosure to extract molecular configuration samples that reflect highly frequent intermolecular interactions from the results of a molecular simulation.

Solution to Problem

According to an aspect, there is provided a structure extraction program that causes a computer to execute a process described below. A plurality of molecular configuration samples each including position information of a plurality of atoms are acquired. Based on distances between pairs of the atoms in the plurality of molecular configuration samples, a score function that calculates a weight for a pair of atoms that belong to different molecules out of the plurality of atoms is generated, the score function including a parameter indicating a distance threshold. A specific value to be set in the parameter is determined based on a change in the weight calculated by the score function in response to a change in a value of the parameter. A priority order of the plurality of molecular configuration samples is determined based on a specific weight calculated by the score function using the specific value and distances between the pairs of atoms in each of the plurality of molecular configuration samples.

According to another aspect, there is provided a structure extraction method executed by a computer. According to another aspect, there is provided an information processing apparatus including a storage unit and a processing unit.

Advantageous Effects of Invention

According to an aspect of the present disclosure, it is possible to extract a molecular configuration sample that reflects highly frequent intermolecular interactions from the results of a molecular simulation.

These and other objects, features, and advantages of the present embodiments will become clear from the following description and accompanying drawings, which are exemplary and explanatory.

Brief Description of Drawings

[FIG. 1] FIG. 1 is useful in explaining an information processing apparatus according to a first embodiment.
[FIG. 2] FIG. 2 is a block diagram depicting example hardware of an information processing apparatus.
[FIG. 3] FIG. 3 depicts one example of a plurality of molecular configuration samples.
[FIG. 4] FIG. 4 depicts one example of statistical information on a physical property.
[FIG. 5] FIG. 5 depicts examples of interatomic distances and cutoff distances.
[FIG. 6] FIG. 6 is a graph indicating example values of a derivative of an average atomic density.
[FIG. 7] FIG. 7 depicts a comparison between experimental results and simulation results.
[FIG. 8] FIG. 8 is a block diagram depicting example functions of an information processing apparatus.
[FIG. 9] FIG. 9 depicts examples of a molecule list and a molecular configuration table.
[FIG. 10] FIG. 10 depicts an example of a score function table.
[FIG. 11] FIG. 11 is a flowchart depicting an example procedure for visualizing a molecular configuration.

Description of Embodiments

Several embodiments will be described below with reference to the accompanying drawings.

First Embodiment

A first embodiment will now be described.

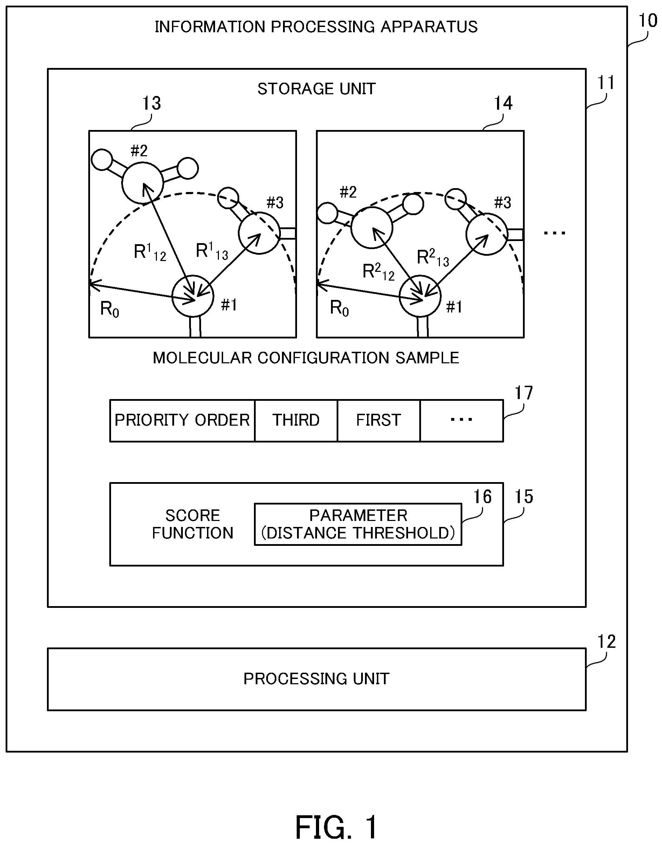

FIG. 1 is useful in explaining an information processing apparatus according to a first embodiment.
An information processing apparatus 10 according to the first embodiment extracts a molecular configuration samples or samples out of a plurality of molecular configuration samples generated by a molecular simulation. This molecular simulation may be executed by the information processing apparatus 10 itself or another information processing apparatus. The information processing apparatus 10 may be a client apparatus or a server apparatus. This information processing apparatus 10 may be referred to as a "computer", a "structure extraction apparatus", or a "simulation apparatus".
The information processing apparatus 10 includes a storage unit 11 and a processing unit 12. The storage unit 11 may be volatile semiconductor memory, such as random access memory (RAM), or may be nonvolatile storage, such as a hard disk drive (HDD) or flash memory. The processing unit 12 is a processor, such as a central processing unit (CPU), a graphics processing unit (GPU), or a digital signal processor (DSP). However, the processing unit 12 may include an electronic circuit, such as an application specific integrated circuit (ASIC) or a field programmable gate array (FPGA). The processor executes a program stored in a memory, such as a RAM (which may be the storage unit 11). A group of processors may be referred to as a "multiprocessor" or simply the "processor".
The storage unit 11 stores a plurality of molecular configuration samples including molecular configuration samples 13 and 14. The molecular configuration samples 13 and 14 indicate the relative positional relationships between two or more molecules, that is, a mutual arrangement of the two or more molecules. The molecular configuration samples 13 and 14 may indicate molecular configurations in a two-dimensional space or may indicate molecular configurations in a three-dimensional space. The molecular configuration samples 13 and 14 may be referred to as "structures" or "three-dimensional structures". The molecular configuration samples 13 and 14 include position information for each of a plurality of atoms contained in two or more molecules. The position information may include two-dimensional coordinates or three-dimensional coordinates.

The molecular configuration samples 13 and 14 are generated through a molecular simulation that analyzes intermolecular interactions. Intermolecular interactions indicate the intermolecular forces by which different molecules attract each other. The molecular simulation may be executed by the information processing apparatus 10 itself or may be executed by another information processing apparatus. As examples, the molecular simulation is a molecular dynamics simulation or a Monte Carlo simulation. In a molecular dynamics simulation, the molecular configuration samples 13 and 14 indicate molecular configurations at different times. In a Monte Carlo simulation, the molecular configuration samples 13 and 14 indicate different molecular configurations that have been randomly generated.

The processing unit 12 generates a score function 15 using the molecular configuration samples 13 and 14. The score function 15 calculates a weight for a pair of atoms belonging to different molecules. The atoms forming this pair may be some of the atoms included in the molecular configuration samples 13 and 14. As one example, some elements, such as hydrogen, may be excluded from the pair. The score function 15 includes a parameter 16 that indicates a distance threshold. This parameter 16 may also be referred to as the "cutoff distance" or "cutoff radius". At this time, the value of the parameter 16 is yet to be optimized. When the value of the parameter 16 changes, the weight calculated by the score function 15 also changes.

The processing unit 12 generates the score function 15 based on the distance between pairs of atoms in the molecular configuration samples 13 and 14. The distance between a given pair of atoms varies according to the molecular configuration sample. The score function 15 calculates a large weight for a pair of atoms that stably remain close to each other. As one example, for a given pair, the score function 15 calculates a weight according to the ratio of molecular configuration samples in which the distance between the pair is equal to or less than the value of the parameter 16. This ratio may be used as the weight.

As another example, the processing unit 12 calculates the value of a potential function for a certain pair from each molecular configuration sample. The potential function indicates the magnitude of an interaction between the atoms. This potential function may depend on the distance between the atoms, and the value of the potential function may decrease as the distance falls. The potential function may depend on the elements of the two atoms, and the value of the potential function may increase when the two atoms are carbon atoms. The score function 15 calculates the weight according to an average of the values of the potential function. This average itself may be used as the weight. However, for molecular configuration samples where the distance between the pair exceeds the value of the parameter 16, the value of the potential function is regarded as zero.

The parameter 16 may be used to determine whether two atoms are close to each other, and may be used to limit the range of a proximity region to be considered when calculating the weight. As one example, when the distance between two atoms exceeds the value of the parameter 16, it is determined that the two atoms are not close. Also, as one example, a mutual arrangement in a molecular configuration sample where the distance exceeds the value of the parameter 16 is not considered when calculating the weight of the two atoms.

As one example, a first atom, a second atom, and a third atom are not hydrogen atoms and belong to different molecules. In the molecular configuration sample 13, the distance between the first atom and the second atom is $R^1_{12}$, and the distance between the first atom and the third atom is $R^1_{13}$. $R^1_{12}$ is greater than $R_0$, and $R^1_{13}$ is equal to or smaller than $R_0$. In the molecular configuration sample 14, the distance between the first atom and the second atom is $R^2_{12}$, and the distance between the first atom and the third atom is $R^2_{13}$. $R^2_{12}$ is equal to or smaller than $R_0$, and $R^2_{13}$ is equal to or smaller than $R_0$.

When the parameter 16 has been set at $R_0$, the score function 15 calculates a weight for a pair of the first atom and the second atom by considering $R^2_{12}$ in the molecular configuration sample 14. The score function 15 also calculates a weight for a pair of the first atom and the third atom by considering $R^1_{13}$ in the molecular configuration sample 13 and $R^2_{13}$ in the molecular configuration sample 14.

The processing unit 12 determines a specific value to be set in the parameter 16 based on a change in the weight of the score function 15 in response to a change in the value of the parameter 16. As one example, the processing unit 12 calculates an evaluation value by dividing the weight of the score function 15 by a spatial size in keeping with the value of the parameter 16 and monitors the change in the evaluation value in response to a change in the value of the parameter 16. When the molecular configuration samples 13 and 14 indicate molecular configurations in a three-dimensional space, the spatial size may be the cube of the value of the parameter 16. When there are a plurality of atom pairs, the evaluation value may be the sum of the weights of such plurality of pairs divided by the spatial size. The processing unit 12 may determine the specific value so that the derivative of the evaluation value with respect to the value of the parameter 16, that is, the unit change in the evaluation value, is maximized.

The processing unit 12 sets the determined specific value, that is, the determined distance threshold, in the parameter 16. The processing unit 12 determines a priority order 17 of the plurality of molecular configuration samples including the molecular configuration samples 13 and 14 based on a specific weight calculated by the score function 15 using the specific value and the distance between the atom pairs in each molecular configuration sample.

As one example, the processing unit 12 calculates a score for each molecular configuration sample based on whether the distance between an atom pair is less than or equal to the value of parameter 16 and the weight calculated by the score function 15, and determines the priority order 17 based on this score. The processing unit 12 may calculate the score using the weight of a pair of atoms whose distance is equal to or less than the value of the parameter 16. When there are a plurality of pairs of atoms, the score may be the sum of the weights of the pairs whose distance is equal to or less than the value of the parameter 16. The processing unit 12 may rank the plurality of molecular configuration samples in descending order of the score. Note that rankings may be assigned to only some of the molecular configuration samples.

The processing unit 12 may generate image data that visualizes position information of atoms included in the molecular configuration samples selected based on the priority order 17 (as one example, one or two or more molecular configuration samples selected in descending order of priority). In this image data, the position of each atom may be represented by a dot, a circle, or a sphere. The processing unit 12 may output the generated image data. As examples, the image data may be displayed on a display apparatus, may be stored in nonvolatile storage, or may be transmitted to another information processing apparatus. This visualization of the molecular configuration may be performed by another information processing apparatus.

As described above, the information processing apparatus 10 according to the first embodiment generates the score function 15 including the parameter 16 indicating a distance threshold based on the distance between a pair of atoms in a plurality of molecular configuration samples. The information processing apparatus 10 determines the specific value to be set in the parameter 16 based on a change in the weight calculated by the score function 15 in response to a change in the value of the parameter 16. The information processing apparatus 10 determines the priority order 17 of a plurality of molecular configuration samples based on the weight calculated by the score function 15 and the distance between a pair of atoms in each molecular configuration sample.

By doing so, a molecular configuration sample or samples that reflect(s) highly frequent intermolecular interactions is extracted from a plurality of molecular configuration samples. Accordingly, compared to other extraction methods, such as extracting the molecular configuration sample that was generated last, molecular configuration samples that are useful for understanding the analysis results of intermolecular interactions produced by a molecular simulation are extracted. The score function 15 is generated and the distance threshold is determined based on the plurality of molecular configuration samples. Accordingly, an appropriate score function 15 that is suited to the present molecular simulation is generated.

As one example, when the distance threshold is too small, there is the risk of the score function 15 insufficiently covering atoms that stably remain close to each other. On the other hand, when the distance threshold is too large, there is the risk of the score function 15 also considering interactions with distant atoms that do not contribute to a stable molecular configuration, which may lower the accuracy of the weight of the score function 15. In this connection, the information processing apparatus 10 is capable of automatically adjusting the distance threshold.

As one example, the magnitude of the interaction and the direction in which two molecules attract each other may differ depending on the molecules being simulated. Also, the simulation conditions, such as water molecules present in the periphery, may differ between molecular simulations. In this connection, the information processing apparatus 10 is capable of calculating an appropriate distance threshold for the present molecular simulation.

The information processing apparatus 10 may generate and output image data that visualizes position information of atoms included in a molecular configuration sample with the highest priority order 17. When doing so, the information processing apparatus 10 may output the image data together with statistical information of physical properties generated from a plurality of molecular configuration samples. By doing so, a visualization result that is consistent with the statistical information, is provided. The information processing apparatus 10 may also generate the score function 15 so as to calculate a weight according to the proportion of molecular configuration samples where the distance between a pair of atoms is equal to or less than the value of the parameter 16. By doing so, it is possible for the score function 15 to evaluate whether a given atom pair stably remain close to each other.

In addition, the information processing apparatus 10 may monitor changes in an evaluation value, which is obtained by dividing the weight of the score function 15 by a spatial size in keeping with the value of the parameter 16, in response to changes in the value of the parameter 16, and determine a value of the parameter 16 so that the derivative of this evaluation value is maximized. By doing so, the information processing apparatus 10 determines the threshold so as to cover a range in which there are many atoms with stable interactions. The information processing apparatus 10 may also calculate a score for each molecular configuration sample based on whether the distance between the atom pairs is equal to or less than the threshold and the weight of the score function 15, and determine the priority order 17 based on such scores. By doing so, it is easy to identify a molecular configuration sample that reflects intermolecular interactions that occur frequently.

Second Embodiment

A second embodiment will now be described.

An information processing apparatus 100 according to the second embodiment analyzes intermolecular interactions by executing a molecular simulation and outputs analysis results. As one example, the information processing apparatus 100 analyzes the intermolecular interactions between a protein and a low-molecular-weight compound to support drug discovery. The molecular simulation referred to here includes molecular dynamics simulations and Monte Carlo simulations. The analysis results include statistical information indicating physical properties, such as interaction energy. The analysis results also include image data that visualizes a typical molecular configuration sample or samples. A typical molecular configuration sample reflects typical intermolecular interactions indicated by the statistical information.

The information processing apparatus 100 may be a client apparatus or may be a server apparatus. The information processing apparatus 100 may be referred to as a "computer", a "structure extraction apparatus", or a "simulation apparatus". The information processing apparatus 100 may execute a structure extraction program. The information processing apparatus 100 corresponds to the information processing apparatus 10 according to the first embodiment.

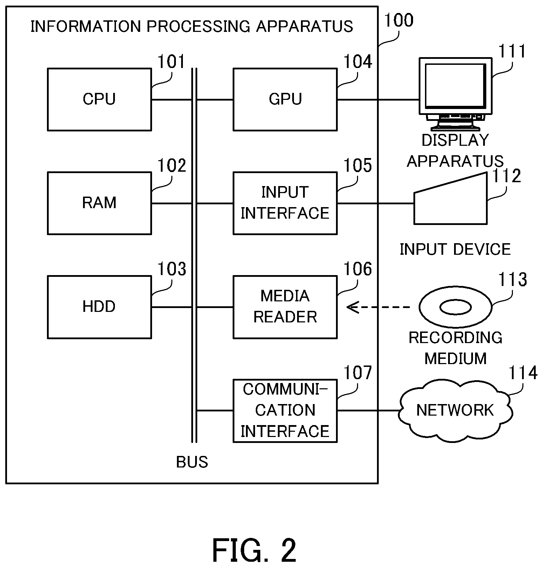

FIG. 2 is a block diagram depicting example hardware of an information processing apparatus.

The information processing apparatus 100 includes a CPU 101, a RAM 102, an HDD 103, a GPU 104, an input interface 105, a media reader 106, and a communication interface 107, which are connected via a bus. The CPU 101 corresponds to the processing unit 12 in the first embodiment. The RAM 102 or the HDD 103 corresponds to the storage unit 11 in the first embodiment.

The CPU 101 is a processor that executes instructions of a program. The CPU 101 loads a program and data stored in the HDD 103 into the RAM 102 and executes the program. The information processing apparatus 100 may include a plurality of processors.

The RAM 102 is a volatile semiconductor memory that temporarily stores a program executed by the CPU 101 and data used in computation by the CPU 101. The information processing apparatus 100 may include another type of volatile memory aside from RAM.

The HDD 103 is nonvolatile storage that stores software programs, such as an operating system (OS), middleware, and application software, in addition to data. The information processing apparatus 100 may also include another type of nonvolatile storage, such as flash memory or a solid state drive (SSD).

The GPU 104 performs image processing in cooperation with the CPU 101 and outputs images to a display apparatus 111 connected to the information processing apparatus 100. As examples, the display apparatus 111 is a cathode ray tube (CRT) display, a liquid crystal display, an organic electroluminescence (EL) display, or a projector. Other types of output device, such as a printer, may be connected to the information processing apparatus 100. The GPU 104 may be used as a general-purpose computing on graphics processing unit (GPGPU). The GPU 104 may execute a program in response to an instruction from the CPU 101. The information processing apparatus 100 may include volatile semiconductor memory aside from the RAM 102 as GPU memory.

The input interface 105 receives an input signal from an input device 112 connected to the information processing apparatus 100. As examples, the input device 112 is a mouse, a touch panel, or a keyboard. A plurality of input devices may be connected to the information processing apparatus 100.

The media reader 106 is a reader apparatus that reads a program and data recorded on a recording medium 113. As examples, the recording medium 113 is a magnetic disk, an optical disc, or a semiconductor memory. Example magnetic disks include flexible disks (FDs) and HDDs. Optical discs include compact discs (CDs) and digital versatile discs (DVDs). The media reader 106 copies a program and data read from the recording medium 113 to another recording medium, such as the RAM 102 or the HDD 103. The program that has been read may be executed by the CPU 101.

The recording medium 113 may be a portable recording medium. The recording medium 113 may be used for distributing a program and data. The recording medium 113 and the HDD 103 may be referred to as "computer-readable recording media".

The communication interface 107 communicates with other information processing apparatuses via a network 114. The communication interface 107 may be a wired communication interface connected to a wired communication apparatus, such as a switch or a router, or a wireless communication interface connected to a wireless communication apparatus, such as a base station or an access point.

Next, molecular simulations will be described.

The information processing apparatus 100 performs a molecular simulation. Such molecular simulation may be applied to various fields, such as life sciences, material development, and drug discovery support. A molecular simulation for drug discovery support may analyze intermolecular interactions between a target protein and a low-molecular-weight compound, or antigen-antibody reactions. In the following description, it is assumed that the information processing apparatus 100 analyzes intermolecular interactions between a protein and a low-molecular-weight compound.

A molecular simulation such as a molecular dynamics simulation or a Monte Carlo simulation generates a large number of molecular configuration samples with different mutual arrangements of two or more molecules. As one example, several thousand to several hundred thousand molecular configuration samples are generated. Since the simulation space in this second embodiment is three-dimensional, the molecular configuration samples may be referred to as three-dimensional structures. The information processing apparatus 100 generates statistical information on physical properties from this plurality of molecular configuration samples. One example of statistical information is the interaction energy between a protein and a low-molecular-weight compound.

The molecular dynamics simulation receives information indicating the elements of the plurality of atoms that form a molecule, and information indicating the initial positions and initial velocities of the plurality of atoms. A molecular dynamics simulation calculates the interaction force that each atom experiences based on the present molecular configuration, and calculates the position and velocity of each atom a small time increment later according to the equation of motion indicated as Math (1). In Math (1), $F_i$ is the interaction force experienced by an atom i, $M_i$ is the mass of the atom i, $R_i$ is the position of the atom i, and t is time. The molecular dynamics simulation iteratively updates the position and velocity of each atom as time advances. The molecular dynamics simulation thereby generates a plurality of molecular configuration samples corresponding to a plurality of times.

$$ F_i = m_i \frac{d^2 r_i}{dt^2} \qquad (1) $$

A Monte Carlo simulation repeatedly generates molecular configuration samples randomly according to a probability distribution indicated by the Boltzmann factor in Math (2). In Math (2), E is the energy of an atomic configuration, $k_B$ is the Boltzmann constant, and T is the temperature. According to the probability distribution indicated by the Boltzmann factor, there is a high probability of generating a stable atomic configuration where the energy E is low and a low probability of generating an unstable atomic configuration where the energy E is high. For this reason, a Monte Carlo simulation generates a plurality of molecular configuration samples corresponding to a plurality of trials.

$$ \exp\left\{ \frac{-E}{k_B T} \right\} \qquad (2) $$

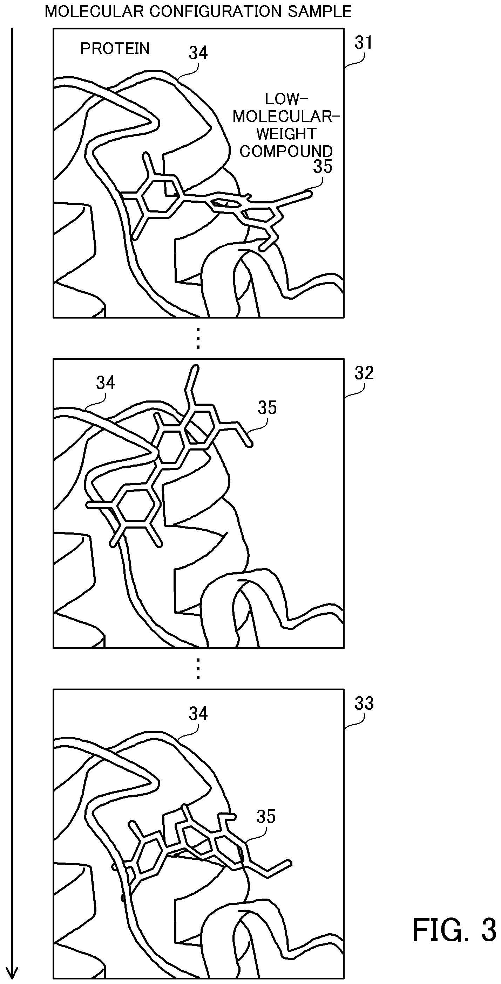

FIG. 3 depicts one example of a plurality of molecular configuration samples.

The molecular configuration samples 31, 32, and 33 are molecular configuration samples corresponding to different times that have been generated by a molecular dynamics simulation. The molecular configuration samples 31, 32, and 33 each indicate the relative positions of a low-molecular-weight compound 35 with respect to a protein 34. However, aside from the protein 34 and the low-molecular-weight compound 35, other molecules, such as water molecules, are also present. As time progresses, the mutual arrangement of the protein 34 and the low-molecular-weight compound 35 will change. Accordingly, the positions of the atoms forming the molecules will differ between the molecular configuration samples 31, 32, and 33.

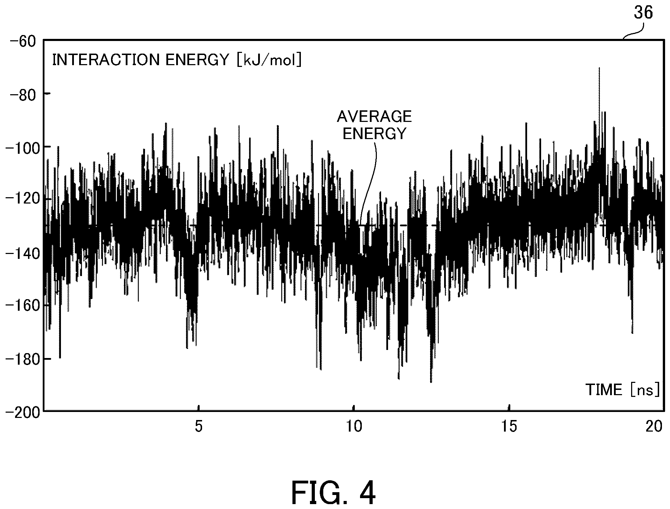

FIG. 4 depicts one example of statistical information on a physical property.

A graph 36 depicts a physical property calculated from molecular configuration samples generated by a molecular dynamics simulation. The horizontal axis of the graph 36 represents time (in units of nanoseconds). The vertical axis of the graph 36 represents the interaction energy (in units of kilojoules/mole) between the protein 34 and the low-molecular-weight compound 35. The interaction energy is calculated from the position of the low-molecular-weight compound 35 relative to the protein 34 according to an equation representing the laws of physics. The mutual arrangement of the protein 34 and the low-molecular-weight compound 35 changes over time, causing the interaction energy to fluctuate. The information processing apparatus 100 may calculate the average energy from the graph 36. The statistical information referred to above may be the interaction energy at each time or may be the average energy.

The information processing apparatus 100 outputs statistical information like that described above as the analysis result of intermolecular interactions. As one example, the information processing apparatus 100 displays the statistical information on the display apparatus 111. When doing so, to assist in understanding the statistical information, the information processing apparatus 100 outputs image data, in which one or a small number of molecular configuration samples are visualized, together with the statistical information. The image data is visual information in which the positions of atoms indicated by the molecular configuration sample are indicated by symbols such as circles. As one example, the information processing apparatus 100 displays this image data on the display apparatus 111.

It is not realistic to visualize and display every molecular configuration sample. For this reason, the information processing apparatus 100 selects representative molecular configuration samples that are useful in understanding the statistical information, out of the large number of molecular configuration samples. However, among the various molecular configuration samples generated by the molecular simulation, some molecular configuration samples do not appropriately represent the trends in the intermolecular interactions indicated by the statistical information. As one example, the molecular configuration indicated by a molecular configuration sample may have occurred by chance and may fail to reflect intermolecular interactions that occur frequently. Visualizing molecular configuration samples that do not reflect frequently occurring intermolecular interactions may make it more difficult to understand the statistical information. It is therefore important for the information processing apparatus 100 to know how to select molecular configuration samples that are representative.

As an example method of selecting a molecular configuration sample, a method that selects the last generated molecular configuration sample is conceivable. However, the suitability of the last molecular configuration sample will depend on the simulation time set for a molecular dynamics simulation and/or the number of iterations set for a Monte Carlo simulation. This means that the final molecular configuration samples do not always reflect intermolecular interactions that occur with high frequency.

As another example method of selecting a molecular configuration sample, a method that calculates a root mean square deviation (RMSD) for the molecular configuration samples and selects the molecular configuration sample located at the center of an RMSD space is also conceivable. RMSD is calculated by calculating the difference in the position coordinates of each atom between two molecular configuration samples and calculating the root mean square of the differences for a plurality of atoms. The molecular configuration sample selected by this method indicates an average molecular configuration out of the molecular configurations that appears in the molecular simulation. However, the molecular configuration sample in the center is susceptible to being influenced by a small number of exceptional molecular configuration samples and may deviate from an ideal molecular configuration sample that reflects the intermolecular interactions that occur with high frequency.

As an example method of selecting molecular configuration samples, one conceivable method uses a score function prepared in advance to calculate the score of each molecular configuration sample and then selects a molecular configuration sample with a high score. As one example of the score, a docking score indicating general binding affinity between molecules could conceivably be used. However, a score function that has been prepared in advance calculates the score from the molecular structure and intermolecular distance objectively and uniquely (that is, as an unchanging calculation), and does not consider the simulation conditions of the molecular simulation, such as the presence of water molecules. Hence, it may fail to calculate appropriate scores that match the statistical information for a plurality of molecular configuration samples.

For this reason, the information processing apparatus 100 dynamically generates the score function from a plurality of molecular configuration samples generated by a molecular simulation, calculates the score for each molecular configuration sample using the generated score function, and selects a molecular configuration sample or samples with a high score.

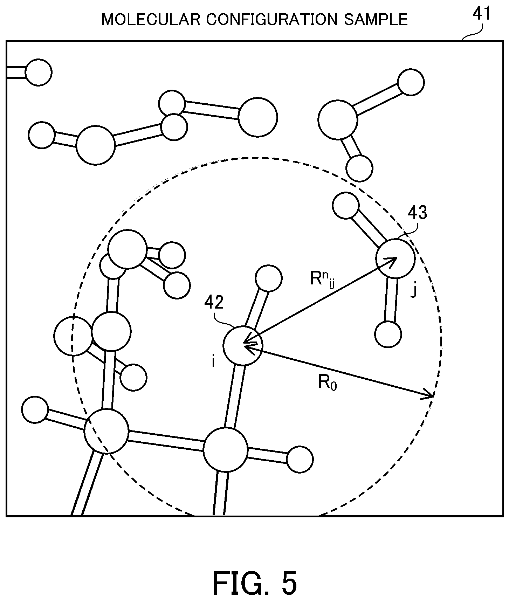

FIG. 5 depicts examples of interatomic distances and cutoff distances.

First, the information processing apparatus 100 generates a score function that calculates the weight of pairs of atoms belonging to different molecules. Such pairs are exhaustively extracted. These pairs include pairs composed of one atom of a protein and one atom of a water molecule, pairs composed of one atom of a low-molecular-weight compound and one atom of a water molecule, pairs composed of one atom of a protein and one atom of a low-molecular-weight compound, and the like. However, hydrogen atoms are excluded from the atoms that form a pair.

The information processing apparatus 100 generates the score function indicated in Math (3) using every molecular configuration sample generated by a molecular simulation. In Math (3), M is the number of molecular configuration samples, $R^n_{ij}$ is the distance between the atoms i and j in the molecular configuration sample n, $R_0$ is the cutoff distance, and $F^{R0}_{ij}$ is the weight of the atoms i and j calculated based on the cutoff distance $R_0$. The cutoff distance $R_0$ is a threshold indicating the range in which intermolecular interactions are considered. This cutoff distance $R_0$ corresponds to the parameter 16 in the first embodiment.

$$F_{ij}^{R0} = \frac{1}{M} \sum_{n}^{M} f\left(R_{ij}^n\right) \delta\left(R_0 - R_{ij}^n\right) \qquad (3)$$

f is a potential function. The potential function f outputs a numerical value indicating the magnitude of the interaction between atoms. The output of the potential function f depends on the interatomic distance and the elements composing the two atoms. The smaller the interatomic distance, the larger the value outputted by the potential function f. When the two atoms are carbon atoms, the potential function f outputs a relatively large value. However, the potential function f may be a function that outputs a constant (as one example, 1). $\delta$ is the delta function. The delta function $\delta$ outputs 0 when an argument is negative and outputs 1 when an argument is non-negative (that is, greater than or equal to 0).

This means that the weight calculated by the score function for the pair of atoms i and j is determined from the cutoff distance $R_0$ and the distances between the atoms i and j in M molecular configuration samples. The weight of the pair of atoms i and j is the average potential obtained by summing the potentials for molecular configuration samples, out of the M molecular configuration samples, where the distance between the atoms i and j is equal to or less than the cutoff distance $R_0$ and then dividing this sum by the number M of the molecular configuration samples. In particular, when the potential function f always outputs 1, the weight of the pair of atoms i and j indicates the proportion of molecular configuration samples in which the distance between the atoms i and j is equal to or less than the cutoff distance $R_0$ out of the M molecular configuration samples.

In a molecular configuration sample 41, an atom 42 corresponds to the atom i, and an atom 43 corresponds to the atom j. The interatomic distance is the distance between the center points of the atoms. The distance $R^n_{ij}$ between the atoms 42 and 43 is equal to or less than the cutoff distance $R_0$. Accordingly, the value of the potential function f for the molecular configuration sample 41 is reflected in the weight of the pair of atoms 42 and 43.

However, the cutoff distance $R_0$ is still unknown when the score function is generated from the molecular configuration samples and is optimized by a method described later. Accordingly, the information processing apparatus 100 defines the score function to output suitable weights for different values of the cutoff distance $R_0$. As one example, the information processing apparatus 100 calculates weights corresponding to various cutoff distances $R_0$, such as the weight when $R_0=3.2$, the weight when $R_0=3.3$, the weight when $R_0=3.4$, and the like.

Next, the information processing apparatus 100 optimizes the cutoff distance $R_0$. At this time, the information processing apparatus 100 calculates an evaluation value s as indicated in Math (4). The evaluation value s for the cutoff distance $R_0$ is calculated by summing the weights for every atom pair <ij> that satisfies the condition described earlier and dividing the sum by the cube of the cutoff distance $R_0$. However, the denominator may be the volume of a sphere whose radius is equal to the cutoff distance $R_0$. When the potential function f always outputs 1, the evaluation value s is proportional to the average atomic density in a sphere of radius $R_0$. As indicated in Math (5), the information processing apparatus 100 calculates the derivative $\Delta s$ of the evaluation value s with respect to the cutoff distance $R_0$.

$$s(R_0) = \frac{1}{R_0^3} \sum_{<ij>} F_{ij}^{R0} \qquad (4)$$

$$\Delta s(R_0) = \frac{ds(R_0)}{dR_0} = \frac{d}{dR_0}\left(\frac{1}{R_0^3} \sum_{<ij>} F_{ij}^{R0}\right) \qquad (5)$$

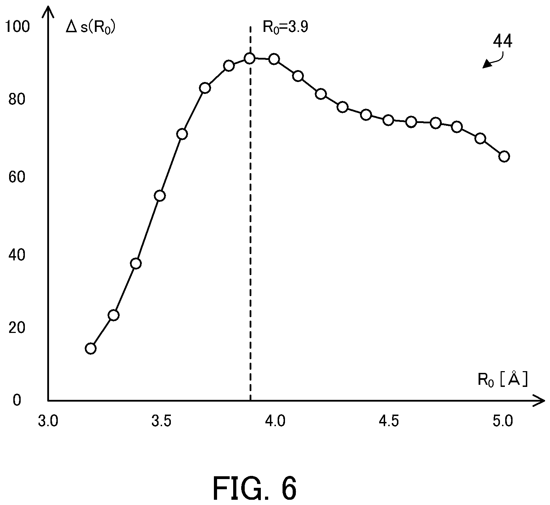

FIG. 6 is a graph indicating example values of the derivative of the average atomic density.

The graph 44 depicts the relationship between the cutoff distance $R_0$ and the derivative $\Delta s$ of the evaluation value. The horizontal axis of graph 44 represents the cutoff distance $R_0$ (in units of angstroms) and the vertical axis of graph 44 represents the derivative $\Delta s$ of the evaluation value corresponding to the cutoff distance $R_0$.

When the cutoff distance $R_0$ is increased in stages, the derivative $\Delta s$ will increase while the cutoff distance $R_0$ is small due to the presence of many partner atoms with stable interactions, that is, many partner atoms with a high probability of being nearby will be located in the periphery. On the other hand, once the cutoff distance $R_0$ becomes sufficiently large, the derivative $\Delta s$ will decrease due to the increase in the number of partner atoms that do not have stable interactions, that is, partner atoms with a low probability of being nearby. For this reason, to enable the score function to determine whether a pair of atoms interact stably, the information processing apparatus 100 selects a cutoff distance $R_0$ at which the derivative $\Delta s$ of the evaluation value is maximized as the optimal value. In the example of graph 44, the information processing apparatus 100 changes the cutoff distance $R_0$ from 3.2 to 5.0 in increments of 0.1 and determines $R_0=3.9$ as the optimal cutoff distance $R_0$.

Next, for each of the plurality of molecular configuration samples, the score indicated in Math (6) is calculated. In Math (6), $R_0$ is the cutoff distance optimized by the method described above, and $S^n$ is the score for a molecular configuration sample n. The score $S^n$ is the sum of the weights of pairs whose distance in the molecular configuration sample n is equal to or less than the cutoff distance $R_0$, out of all of the pairs of atoms <ij> that satisfy the condition described earlier. The information processing apparatus 100 sorts the plurality of molecular configuration samples in descending order of the score $S^n$, selects a predetermined number of, that is, one or two or more, molecular configuration samples in descending order of the score $S^n$, and visualizes the selected molecular configuration sample(s).

$$S^n = \sum_{<ij>} F_{ij}^{R0} \delta(R_0 - R_{ij}^n) \qquad (6)$$

In the selection method of the molecular configuration samples in this second embodiment, the weights of pairs of atoms that are frequently close to each other in all of the molecular configuration samples increase. The score of a molecular configuration sample in which a pair of atoms with a large weight are actually close to each other also increases. Accordingly, a molecular configuration sample that reflects intermolecular interactions that appear with a high frequency is selected. The molecular configuration sample(s) to be visualized is/are uniquely determined based on the generated plurality of molecular configuration samples.

In addition, the cutoff distance is automatically optimized and does not depend on an input from the user. Here, when an inappropriate cutoff distance is used, there is the risk of an inappropriate score function being generated and the risk of an inappropriate molecular configuration sample being selected. When the cutoff distance is too small, the range of partner atoms whose interactions are examined is too narrow, which may result in a score function that does not properly evaluate whether an atom pair has a stable interaction. On the other hand, when the cutoff distance is too large, the score function will evaluate interactions with distant partner atoms that do not contribute to stabilization of the molecular configuration, which may cause noise in the weight of the score function.

The appropriate cutoff distance also differs according to the molecular simulation. Different types of protein and/or low-molecular-weight compound have different types of interacting atom pairs and/or mutual arrangements of the atom pairs. Also, when the initial position or initial orientation of a low-molecular-weight compound differs, the low-molecular-weight compound may bind to a different part of the protein and may bind to the protein with a different orientation. In contrast, with the method of selecting molecular configuration samples according to the second embodiment, an appropriate cutoff distance for the molecular simulation being performed is calculated.

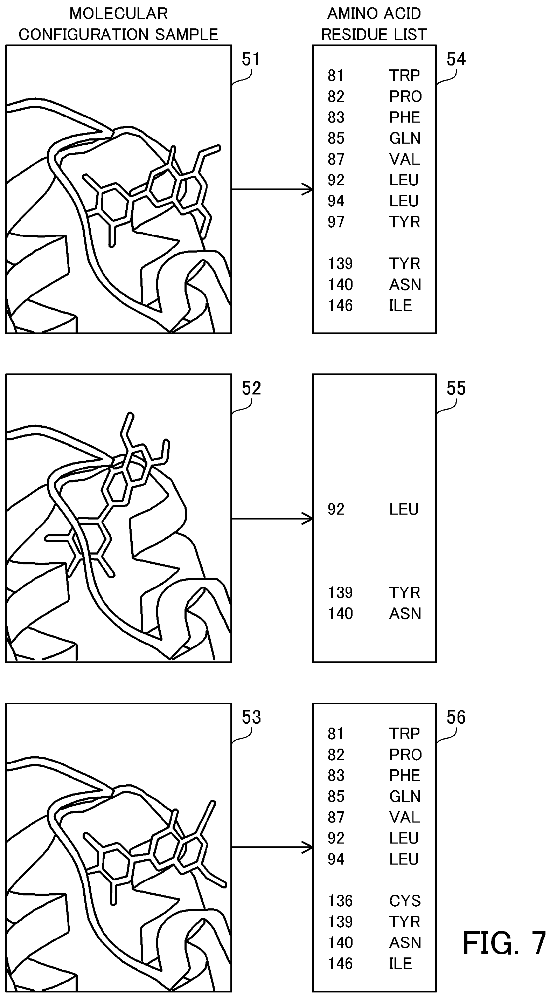

FIG. 7 depicts a comparison between experimental results and simulation results.

A molecular configuration sample 51 depicts the correct molecular configuration that has been measured by a chemical experiment and X-ray crystal structure analysis. A molecular configuration sample 52 is the molecular configuration sample with the lowest score out of the molecular configuration samples generated by a molecular simulation. A molecular configuration sample 53 is the molecular configuration sample with the highest score.

An amino acid residue list 54 indicates amino acid residues that are within the cutoff distance $R_0$ from the low-molecular-weight compound in the molecular configuration sample 51. An amino acid residue list 55 indicates amino acid residues that are within the cutoff distance $R_0$ from the low-molecular-weight compound in the molecular configuration sample 52. An amino acid residue list 56 indicates amino acid residues that are within the cutoff distance $R_0$ from the low-molecular-weight compound in the molecular configuration sample 53.

An amino acid residue is a part corresponding to one amino acid unit contained in a protein. The amino acid residue lists 54, 55, and 56 each include position numbers indicating positions in the protein and the names of amino acid residues. "TRP" is tryptophan, "PRO" is proline, "PHE" is phenylalanine, "GLN" is glutamine, "VAL" is valine, "LEU" is leucine, "TYR" is tyrosine, "CYS" is cysteine, "ASN" is asparagine, and "ILE" is isoleucine.

The amino acid residue list 54 includes eleven amino acid residues. The amino acid residue list 55 includes three amino acid residues, out of the eleven amino acid residues indicated in the amino acid residue list 54. The amino acid residue list 56 includes ten amino acid residues out of the eleven amino acid residues indicated in the amino acid residue list 54. Accordingly, the molecular configuration sample 53 with the highest score reproduces the measurement results produced by experimentation with high accuracy.

Next, the functions and processing procedure of the information processing apparatus 100 will be described.

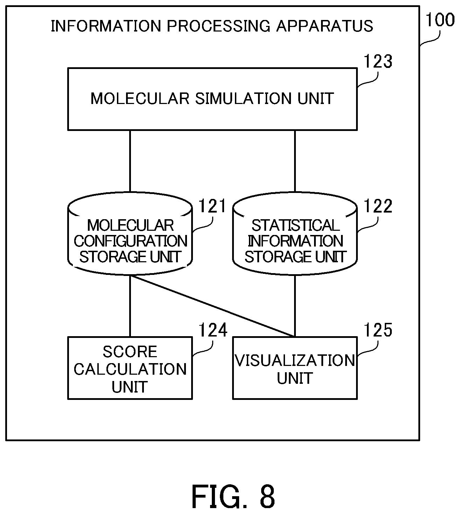

FIG. 8 is a block diagram depicting example functions of the information processing apparatus.

The information processing apparatus 100 includes a molecular configuration storage unit 121, a statistical information storage unit 122, a molecular simulation unit 123, a score calculation unit 124, and a visualization unit 125. The molecular configuration storage unit 121 and the statistical information storage unit 122 are implemented using the RAM 102 or the HDD 103, for example. The molecular simulation unit 123, the score calculation unit 124, and the visualization unit 125 are implemented using the CPU 101 and a program, for example.

The molecular configuration storage unit 121 stores definitions of molecules to be placed in a simulation space. The molecular configuration storage unit 121 also stores a plurality of molecular configuration samples generated by a molecular simulation. Each molecular configuration sample includes the position coordinates of each of a plurality of atoms. For a molecular dynamics simulation, the molecular configuration storage unit 121 stores a molecular configuration sample of an initial arrangement and a molecular configuration sample at each point of time. For a Monte Carlo simulation, the molecular configuration storage unit 121 stores molecular configuration samples that have been randomly generated.

The statistical information storage unit 122 stores statistical information on a physical property generated by a molecular simulation. As one example, the statistical information storage unit 122 stores at least one of interaction energy and average energy at each time indicated in FIG. 4 as the statistical information.

The molecular simulation unit 123 executes a molecular simulation based on the definition of a molecule stored in the molecular configuration storage unit 121. As examples, the molecular simulation unit 123 executes a molecular dynamics simulation or a Monte Carlo simulation. The molecular simulation unit 123 stores a plurality of molecular configuration samples in the molecular configuration storage unit 121. In addition, the molecular simulation unit 123 generates statistical information of one or more physical properties from the plurality of molecular configuration samples and stores the statistical information in the statistical information storage unit 122.

The score calculation unit 124 generates a score function using a plurality of molecular configuration samples stored in the molecular configuration storage unit 121. The score calculation unit 124 optimizes the cutoff distance so as to maximize the derivative of the evaluation value and calculates the score of each molecular configuration sample using the score function and the optimized cutoff distance.

The visualization unit 125 selects a predetermined number of, that is, one or two or more, molecular configuration samples with high scores out of the plurality of molecular configuration samples. The visualization unit 125 generates image data that visualizes the mutual arrangement of molecules indicated by the selected molecular configuration samples. The visualization unit 125 associates the statistical information stored in the statistical information storage unit 122 with the generated image data and outputs the associated information. The visualization unit 125 may display the statistical information and the image data on the display apparatus 111, may store both in nonvolatile storage, and/or may transmit both to another information processing apparatus.

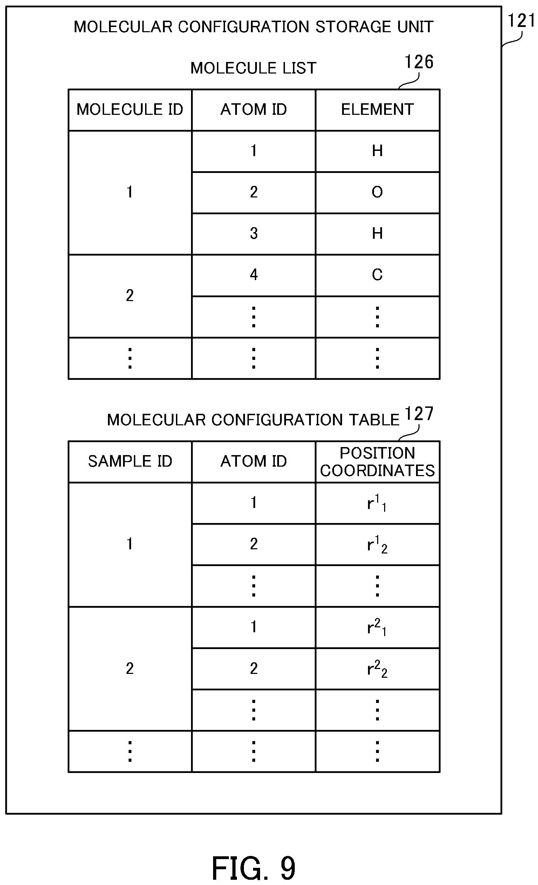

FIG. 9 depicts examples of a molecule list and a molecular configuration table.

A molecule list 126 is stored in the molecular configuration storage unit 121. The molecule list 126 associates molecule IDs, atom IDs, and elements. The molecule ID is an identifier that identifies molecules, such as proteins, low-molecular-weight compounds, and water molecules. The atom ID is an identifier for identifying an atom forming a molecule. The element is a type of atom, such as hydrogen (H), oxygen (O), or carbon (C). Note that the molecular configuration storage unit 121 also stores information indicating the connection relationships between a plurality of atoms in the same molecule.

A molecular configuration table 127 is stored in the molecular configuration storage unit 121. The molecular configuration table 127 associates a sample ID, an atom ID, and position coordinates. The sample ID is an identifier that identifies a molecular configuration sample. The atom ID is an identifier that identifies an atom. The position coordinates are three-dimensional coordinates that indicate the position of an atom in a molecular configuration sample.

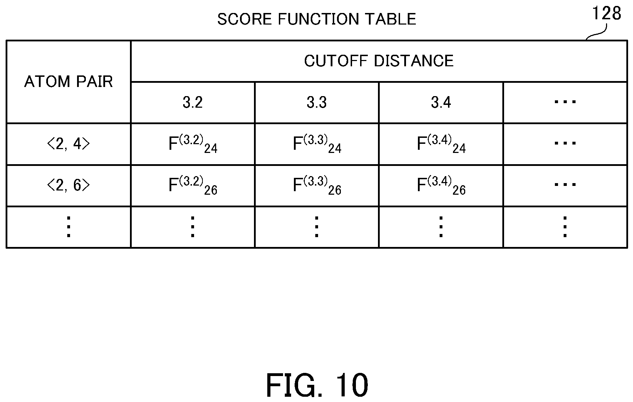

FIG. 10 depicts an example of a score function table.

A score function table 128 is generated by the score calculation unit 124 and stored in the RAM 102. The score function table 128 indicates the score function before the cutoff distance has been optimized. The score function table 128 stores weights, which have been calculated for each of a plurality of cutoff distances, in association with two atom IDs indicating an atom pair.

As examples, the score function table 128 lists a plurality of weights for a pair of a second atom and a fourth atom, such as a weight when $R_0=3.2$, a weight when $R_0=3.3$, and a weight when $R_0=3.4$. In the same way, the score function table 128 lists a plurality of weights for a pair of the second atom and a sixth atom, such as a weight when $R_0=3.2$, a weight when $R_0=3.3$, and a weight when $R_0=3.4$.

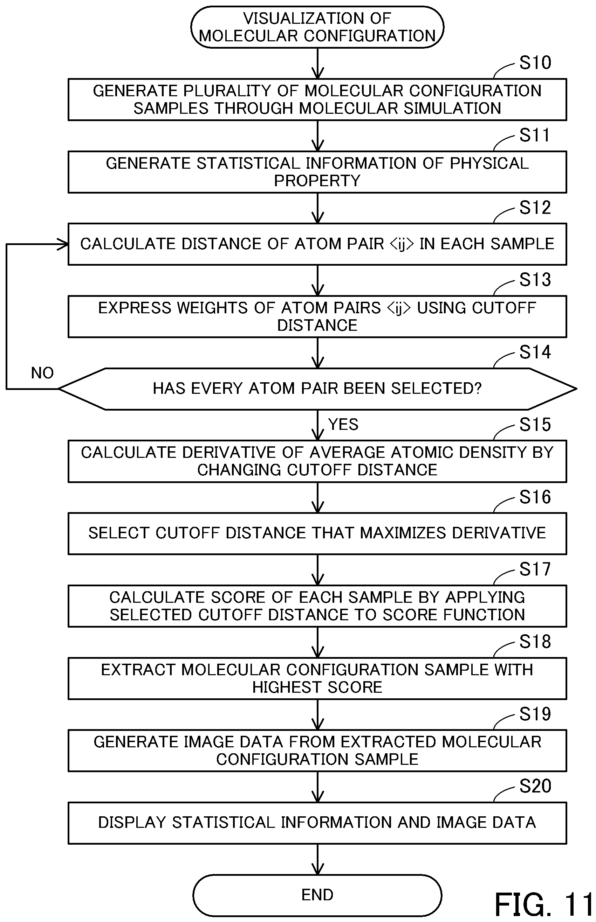

FIG. 11 is a flowchart depicting an example procedure for visualizing a molecular configuration.

(S10) The molecular simulation unit 123 generates M molecular configuration samples, in each of which at least some of the atoms are at different positions, by way of a molecular simulation. As one example, the molecular simulation unit 123 generates M molecular configuration samples corresponding to different times by way of a molecular dynamics simulation. As another example, the molecular simulation unit 123 randomly generates M molecular configuration samples by way of a Monte Carlo simulation.

(S11) The molecular simulation unit 123 generates statistical information on a physical property or properties from the M molecular configuration samples generated in step S10. As one example, the molecular simulation unit 123 calculates the interaction energy between a protein and a low-molecular-weight compound.

(S12) The score calculation unit 124 selects a pair of atoms (that is, the atoms i and j) that are not hydrogen atoms and belong to different molecules. The score calculation unit 124 calculates the distance between the atoms i and j in each of the M molecular configuration samples.

(S13) The score calculation unit 124 substitutes the M distances calculated in step S12 into Math (3) and expresses weights of the pair of atoms i and j using cutoff distances. As one example, the score calculation unit 124 calculates a weight corresponding to each candidate for the cutoff distance.

(S14) The score calculation unit 124 determines whether every atom pair has been selected in step S12. When every atom pair has been selected, the processing proceeds to step S15, but when there is an unselected atom pair, the processing returns to step S12.

(S15) The score calculation unit 124 calculates an evaluation value indicating an average atomic density by dividing the sum of the weights of all of the atom pairs by the cube of the cutoff distance. The score calculation unit 124 calculates the derivative of the evaluation value while increasing the cutoff distance in fixed increments. As one example, the derivative of the evaluation value is simply calculated by dividing the difference between the evaluation value corresponding to a certain cutoff distance and the evaluation value corresponding to the immediately preceding cutoff distance by the increase in the cutoff distance.

(S16) The score calculation unit 124 selects, as an optimal value, the cutoff distance at which the derivative of the evaluation value calculated in step S15 is maximized.

(S17) The score calculation unit 124 applies the cutoff distance selected in step S16 to the score function. The score calculation unit 124 calculates the scores of the M molecular configuration samples using this score function in accordance with Math (6).

(S18) The visualization unit 125 extracts the molecular configuration sample with the highest score. However, the visualization unit 125 may extract two or more molecular configuration samples in order from the highest score.

(S19) The visualization unit 125 generates image data that visualizes the molecular configuration sample(s) extracted in step S18. As one example, the visualization unit 125 generates image data in which atoms are represented by dots, circles, or spheres in accordance with the position coordinates indicated by the extracted molecular configuration sample(s).

(S20) The visualization unit 125 displays the statistical information generated in step S11 in association with the image data generated in step S19.

As described above, the information processing apparatus 100 according to the second embodiment selects and visualizes one or more molecular configuration samples out of a large number of molecular configuration samples generated by a molecular simulation and displays the selected samples together with statistical information. By doing so, it is easy for the user to understand statistical information relating to intermolecular interactions. The information processing apparatus 100 calculates a score for each of a plurality of molecular configuration samples and selects a molecular configuration sample or samples with a high score. By doing so, a molecular configuration sample that matches the statistical information is selected, compared to a case where the last generated molecular configuration sample or a randomly selected molecular configuration sample is visualized.

The information processing apparatus 100 also uses the plurality of molecular configuration samples generated by the present molecular simulation to generate a score function and optimize the cutoff distance. By doing so, compared to when a fixed score function or a fixed cutoff distance is used, an appropriate score function that is suitable for the present molecular simulation is generated and pairs of atoms that contribute to intermolecular interactions are appropriately identified. The information processing apparatus 100 calculates the score of a molecular configuration sample by summing the weights of the pairs of atoms whose distance is equal to or less than the cutoff distance. By doing so, a molecular configuration sample that reflects highly frequent intermolecular interactions is selected.

In addition, the information processing apparatus 100 calculates an evaluation value indicating the average atomic density by dividing the sum of weights of various atom pairs by a unit space size corresponding to the cutoff distance and determines the cutoff distance so as to maximize the derivative of this evaluation value. By doing so, the cutoff distance is determined so that the score function covers a range with many atoms with stable interactions, and pairs of atoms that contribute to highly frequent intermolecular interactions are appropriately evaluated.

The explanation given above merely describes the principles of the present embodiments. A large number of changes and modifications could be made by one skilled in the art, so that the present embodiments are not limited to the specifically recited configurations and example applications described herein. All of such modifications and their equivalents are to be regarded as being included within the scope of the embodiments as defined by the attached claims and their equivalents.

Reference Signs List

| 10 | Information processing apparatus |
| 11 | Storage unit |
| 12 | Processing unit |
| 13, 14 | Molecular configuration sample |
| 15 | Score function |
| 16 | Parameter |
| 17 | Priority order |

**Claims**

**1.** A structure extraction program that causes a computer to execute a process comprising:

acquiring a plurality of molecular configuration samples each including position information of a plurality of atoms;
generating, based on distances between pairs of the atoms in the plurality of molecular configuration samples, a score function that calculates a weight for a pair of atoms that belong to different molecules out of the plurality of atoms, the score function including a parameter indicating a distance threshold;
determining a specific value to be set in the parameter, based on a change in the weight calculated by the score function in response to a change in a value of the parameter; and
determining a priority order of the plurality of molecular configuration samples, based on a specific weight calculated by the score function using the specific value and distances between the pairs of atoms in each of the plurality of molecular configuration samples.

2. The structure extraction program according to claim 1,
wherein the score function calculates the weight in keeping with a ratio of molecular configuration samples in which the distance between the pair of atoms is equal to or less than the value of the parameter, out of the plurality of molecular configuration samples.

3. The structure extraction program according to claim 1,
wherein the determining of the specific value includes monitoring a change in an evaluation value obtained by dividing the weight calculated by the score function by a spatial size corresponding to the value of the parameter in response to the change in the value of the parameter, and determining the specific value so that a derivative of the evaluation value is maximized.

4. The structure extraction program according to claim 1,
wherein the determining of the priority order includes calculating a score for each of the plurality of molecular configuration samples, based on the specific weight and on whether the distance between the pair of atoms is equal to or less than the specific value, and determining the priority order based on the score.

5. The structure extraction program according to claim 1,
wherein the process further includes:

generating image data, which visualizes position information of the plurality of atoms included in a molecular configuration sample with a highest priority order out of the plurality of molecular configuration samples; and outputting the image data.

6. A structure extraction method executed by a computer, the structure extraction method comprising:

acquiring a plurality of molecular configuration samples each including position information of a plurality of atoms;
generating, based on distances between pairs of the atoms in the plurality of molecular configuration samples, a score function that calculates a weight for a pair of atoms that belong to different molecules out of the plurality of atoms, the score function including a parameter indicating a distance threshold;
determining a specific value to be set in the parameter, based on a change in the weight calculated by the score function in response to a change in a value of the parameter; and
determining a priority order of the plurality of molecular configuration samples, based on a specific weight calculated by the score function using the specific value and distances between the pairs of atoms in each of the plurality of molecular configuration samples.

7. An information processing apparatus comprising:

a storage unit that stores a plurality of molecular configuration samples each including position information of a plurality of atoms; and
a processing unit that generates, based on distances between pairs of the atoms in the plurality of molecular configuration samples, a score function that calculates a weight for a pair of atoms that belong to different molecules out of the plurality of atoms, the score function including a parameter indicating a distance threshold, determines a specific value to be set in the parameter, based on a change in the weight calculated by the score function in response to a change in a value of the parameter, and determines a priority order of the plurality of molecular configuration samples, based on a specific weight calculated by the score function using the specific value and distances between the pairs of atoms in each of the plurality of molecular configuration samples.

FIG. 1

INFORMATION PROCESSING APPARATUS 100

CPU 101

GPU 104

DISPLAY APPARATUS 111

RAM 102

INPUT INTERFACE 105

INPUT DEVICE 112

HDD 103

MEDIA READER 106

RECORDING MEDIUM 113

COMMUNI-CATION INTERFACE 107

NETWORK 114

BUS

FIG. 2

MOLECULAR CONFIGURATION SAMPLE

FIG. 3

FIG. 4

MOLECULAR CONFIGURATION SAMPLE 41

FIG. 5

FIG. 6

MOLECULAR
CONFIGURATION SAMPLE

AMINO ACID
RESIDUE LIST

51

54

| 81 | TRP |
| 82 | PRO |
| 83 | PHE |
| 85 | GLN |
| 87 | VAL |
| 92 | LEU |
| 94 | LEU |
| 97 | TYR |
| | |
| 139 | TYR |
| 140 | ASN |
| 146 | ILE |

52

55

| 92 | LEU |
| | |
| 139 | TYR |
| 140 | ASN |

53

56

| 81 | TRP |
| 82 | PRO |
| 83 | PHE |
| 85 | GLN |
| 87 | VAL |
| 92 | LEU |
| 94 | LEU |
| | |
| 136 | CYS |
| 139 | TYR |
| 140 | ASN |
| 146 | ILE |

FIG. 7

INFORMATION PROCESSING APPARATUS 100

MOLECULAR SIMULATION UNIT 123

MOLECULAR CONFIGURATION STORAGE UNIT 121

STATISTICAL INFORMATION STORAGE UNIT 122

SCORE CALCULATION UNIT 124

VISUALIZATION UNIT 125

# FIG. 8

MOLECULAR CONFIGURATION STORAGE UNIT — 121

MOLECULE LIST — 126

| MOLECULE ID | ATOM ID | ELEMENT |
|---|---|---|
| 1 | 1 | H |
| | 2 | O |
| | 3 | H |
| 2 | 4 | C |
| | ⋮ | ⋮ |
| ⋮ | ⋮ | ⋮ |

MOLECULAR CONFIGURATION TABLE — 127

| SAMPLE ID | ATOM ID | POSITION COORDINATES |
|---|---|---|
| 1 | 1 | $r^1_1$ |
| | 2 | $r^1_2$ |
| | ⋮ | ⋮ |
| 2 | 1 | $r^2_1$ |
| | 2 | $r^2_2$ |
| | ⋮ | ⋮ |
| ⋮ | ⋮ | ⋮ |

FIG. 9

SCORE FUNCTION TABLE 128

| ATOM PAIR | CUTOFF DISTANCE | | | |
|---|---|---|---|---|
| | 3.2 | 3.3 | 3.4 | ⋯ |
| $\langle 2, 4\rangle$ | $F^{(3.2)}{}_{24}$ | $F^{(3.3)}{}_{24}$ | $F^{(3.4)}{}_{24}$ | ⋯ |
| $\langle 2, 6\rangle$ | $F^{(3.2)}{}_{26}$ | $F^{(3.3)}{}_{26}$ | $F^{(3.4)}{}_{26}$ | ⋯ |
| ⋮ | ⋮ | ⋮ | ⋮ | ⋮ |

# FIG. 10

```
        ┌─────────────────────────────┐
        │      VISUALIZATION OF       │
        │   MOLECULAR CONFIGURATION   │
        └─────────────────────────────┘
                      │
                      ▼                          S10
   ┌─────────────────────────────────────────────┐
   │  GENERATE PLURALITY OF MOLECULAR CONFIGURATION │
   │    SAMPLES THROUGH MOLECULAR SIMULATION       │
   └─────────────────────────────────────────────┘
                      │
                      ▼                          S11
   ┌─────────────────────────────────────────────┐
   │   GENERATE STATISTICAL INFORMATION OF PHYSICAL │
   │                 PROPERTY                       │
   └─────────────────────────────────────────────┘
                      │
                      ▼                          S12
   ┌─────────────────────────────────────────────┐
   │  CALCULATE DISTANCE OF ATOM PAIR <ij> IN EACH SAMPLE │
   └─────────────────────────────────────────────┘
                      │
                      ▼                          S13
   ┌─────────────────────────────────────────────┐
   │  EXPRESS WEIGHTS OF ATOM PAIRS <ij> USING CUTOFF │
   │                 DISTANCE                       │
   └─────────────────────────────────────────────┘
                      │
                      ▼                          S14
   NO  ◁─────  HAS EVERY ATOM PAIR BEEN SELECTED?  ▷
                      │ YES                       S15
                      ▼
   ┌─────────────────────────────────────────────┐
   │  CALCULATE DERIVATIVE OF AVERAGE ATOMIC DENSITY BY │
   │          CHANGING CUTOFF DISTANCE              │
   └─────────────────────────────────────────────┘
                      │
                      ▼                          S16
   ┌─────────────────────────────────────────────┐
   │   SELECT CUTOFF DISTANCE THAT MAXIMIZES DERIVATIVE │
   └─────────────────────────────────────────────┘
                      │
                      ▼                          S17
   ┌─────────────────────────────────────────────┐
   │     CALCULATE SCORE OF EACH SAMPLE BY APPLYING │
   │  SELECTED CUTOFF DISTANCE TO SCORE FUNCTION    │
   └─────────────────────────────────────────────┘
                      │
                      ▼                          S18
   ┌─────────────────────────────────────────────┐
   │   EXTRACT MOLECULAR CONFIGURATION SAMPLE WITH  │
   │               HIGHEST SCORE                    │
   └─────────────────────────────────────────────┘
                      │
                      ▼                          S19
   ┌─────────────────────────────────────────────┐
   │  GENERATE IMAGE DATA FROM EXTRACTED MOLECULAR  │
   │          CONFIGURATION SAMPLE                  │
   └─────────────────────────────────────────────┘
                      │
                      ▼                          S20
   ┌─────────────────────────────────────────────┐
   │  DISPLAY STATISTICAL INFORMATION AND IMAGE DATA │
   └─────────────────────────────────────────────┘
                      │
                      ▼
        ┌─────────────────────────────┐
        │             END             │
        └─────────────────────────────┘
```

FIG. 11

| | |
|---|---|
| **INTERNATIONAL SEARCH REPORT** | International application No. |
| | **PCT/JP2022/019088** |

**A. CLASSIFICATION OF SUBJECT MATTER**

*G16C 20/10*(2019.01)i; *G16C 20/80*(2019.01)i
FI:    G16C20/10; G16C20/80

According to International Patent Classification (IPC) or to both national classification and IPC

**B. FIELDS SEARCHED**

Minimum documentation searched (classification system followed by classification symbols)

G16C20/10; G16C20/80

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 1989-84392 A (DAIKIN IND LTD) 29 March 1989 (1989-03-29)<br>entire text, all drawings | 1-7 |
| A | JP 2007-219789 A (NEC CORP) 30 August 2007 (2007-08-30)<br>entire text, all drawings | 1-7 |
| A | JP 2008-158781 A (NEC CORP) 10 July 2008 (2008-07-10)<br>entire text, all drawings | 1-7 |
| A | JP 2015-230535 A (JAPAN AGENCY FOR MARINE-EARTH SCIENCE AND<br>TECHNOLOGY) 21 December 2015 (2015-12-21)<br>entire text, all drawings | 1-7 |
| A | JP 2008-269443 A (FUJI XEROX CO LTD) 06 November 2008 (2008-11-06)<br>entire text, all drawings | 1-7 |

☐ Further documents are listed in the continuation of Box C.　　☑ See patent family annex.

| | | |
|---|---|---|
| \* | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **20 July 2022** | **09 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/019088**

| Patent document cited in search report | | | Publication date (day/month/year) | Patent family member(s) | Publication date (day/month/year) |
|---|---|---|---|---|---|
| JP | 1989-84392 | A | 29 March 1989 | (Family: none) | |
| JP | 2007-219789 | A | 30 August 2007 | (Family: none) | |
| JP | 2008-158781 | A | 10 July 2008 | (Family: none) | |
| JP | 2015-230535 | A | 21 December 2015 | US 2017/0193251 A1 entire text, all drawings EP 3153982 A1 | |
| JP | 2008-269443 | A | 06 November 2008 | (Family: none) | |

**REFERENCES CITED IN THE DESCRIPTION**

**Patent documents cited in the description**

- JP 2006190234 A

- WO 2009034297 A